# EUROPEAN PATENT APPLICATION

(11) **EP 4 625 243 A1**
(43) Date of publication of application: **01.10.2025**
(21) Application number: 25165843.1
(22) Date of filing: 25.03.2025
(51) Int. Cl.: G06F 40/30, G16H 10/60, G16H 50/20

(54) **SYSTEM AND METHOD FOR DETERMINING STRUCTURED DATA**

(30) Priority: 27.03.2024 US 202463570581 P
(71) Applicant: Solventum Intellectual Properties Company, Maplewood, MN 55144 (US)
(72) Inventor: Nuno, Kazuma, Saint Paul, 55101 (US); Reed, Emma, Pittsburgh, 15221 (US); Maimon, Shir, Pittsburgh, 15232 (US); Lines, Jennifer, Pittsburgh, 15218 (US)
(74) Representative: Vossius & Partner Patentanwälte Rechtsanwälte mbB

(57) **Abstract**

A system for determining structured data includes a processor configured to receive a plurality of medical concepts and a corresponding plurality of labels. The processor is further configured to receive a transcript of a conversation between a physician and a patient. The processor is further configured to determine a plurality of transcript concepts in the transcript based on the plurality of medical concepts. The processor is further configured to assign each transcript concept with the label associated with the corresponding medical concept. The processor is further configured to determine a plurality of concept combinations by combining the plurality of transcript concepts. The processor is further configured to determine, via a machine learning model, a combination label for each concept combination. The combination label is a valid label or invalid label. The processor is further configured to generate an output structured data based on the concept combinations having the valid label.

## Description

### Technical Field

The present disclosure generally relates to a system and a method for determining structured data.

### Background

Conversations between patients and medical practitioners, such as doctors and nurses, are often recorded. The record of the conversation (e.g., a clinical note), and a transcript, are a part of the patient's medical record data. Generally, the transcript may be generated by automatic speech recognition or by a trained (human) medical transcriptionist listening to a recording of the conversation.

In recent years, extensive research and development effort in healthcare industry has been focused on automating a process of generating the clinical note based on doctor-patient conversations (audio or transcript). Typically, in case of doctor-patient conversation transcripts, pieces of information (e.g., words, phrases, etc.) may need to be extracted from the transcript. Such pieces of information may be combined with other pieces to form a target structed data. Structured data is data organized into specific fields (or categories) as part of a schema, with each field having a defined purpose. Examples of such fields may include numbers, anatomical structures, laterality, etc. Structured data categories may include patient history, family history, past surgeries, medications, exam results, vital signs, and more.

Automation of such extraction improves both accuracy and efficiency of documentation processes, such as electronic medical record (EMR) input and clinical note generation. Common challenges in automated extraction of structured data from conversational text may include variety in vocabularies and sentence structures in the text, presence of medically irrelevant information, noise such as fillers, garbles, restating, repeating, rephrasing, etc., implicitly or contextually assumed information, unreliable sentence delimitation by the upstream automatic speech recognition, and pieces of information relevant to a single structured data instance but mentioned apart from each other.

### Summary

In a first aspect, the present disclosure provides a system for determining structured data. The system includes at least one non-transitory computer-readable storage medium having instructions stored thereon. The system further includes at least one computer processor coupled to the at least one non-transitory computer-readable storage medium and configured to execute the instructions to receive a plurality of medical concepts and a corresponding plurality of labels. Each medical concept from the plurality of medical concepts is associated with a corresponding label from the plurality of labels. The computer processor is further configured to receive a plurality of target attributes associated with a plurality of fields of one or more structured data. Each field from the plurality of fields is associated with one or more target attributes from the plurality target of attributes. The computer processor is further configured to receive a transcript of a conversation between a physician and a patient. The computer processor is further configured to determine a plurality of transcript concepts in the transcript based on the plurality of medical concepts. Each transcript concept from the plurality of transcript concepts is a corresponding medical concept from the plurality of medical concepts. The computer processor is further configured to assign each transcript concept with the label associated with the corresponding medical concept. The computer processor is further configured to determine a plurality of concept combinations by combining the plurality of transcript concepts. Each concept combination from the plurality of concept combinations is a combination of two or more transcript concepts from the plurality of transcript concepts, such that the labels of the two or more transcript concepts associate with one or more target attributes from the plurality of target attributes of a same field from the plurality of fields. The computer processor is further configured to determine, via a machine learning model, a combination label for each concept combination. The combination label is a valid label or an invalid label. The computer processor is further configured to generate an output structured data based on the concept combinations having the valid label.

In a second aspect, the present disclosure provides a method for determining structured data. The method includes receiving, via at least one computer processor, a plurality of medical concepts and a corresponding plurality of labels. Each medical concept from the plurality of medical concepts is associated with a corresponding label from the plurality of labels. The method further includes receiving, via the at least one computer processor, a plurality of target attributes associated with a plurality of fields of one or more structured data. Each field from the plurality of fields is associated with one or more target attributes from the plurality of target attributes. The method further includes receiving, via the at least one computer processor, a transcript of a conversation between a physician and a patient. The method further includes determining, via the at least one computer processor, a plurality of transcript concepts in the transcript based on the plurality of medical concepts. Each transcript concept from the plurality of transcript concepts is a corresponding medical concept from the plurality of medical concepts. The method further includes assigning, via the at least one computer processor, each transcript concept with the label associated with the corresponding medical concept. The method further includes determining, via the at least one computer processor, a plurality of concept combinations by combining the plurality of transcript concepts. Each concept combination from the plurality of concept combinations is a combination of two or more transcript concepts from the plurality of transcript concepts, such that the labels of the two or more transcript concepts associate with one or more target attributes from the plurality of target attributes of a same field from the plurality of fields. The method further includes determining, via a machine learning model, a combination label for each concept combination. The combination label is a valid label or an invalid label. The method further includes generating, via the at least one computer processor, an output structured data based on the concept combinations having the valid label.

The details of one or more examples of the disclosure are set forth in the accompanying drawings and the description below. Other features, objects, and advantages of the disclosure will be apparent from the description and drawings, and from the claims.

### Brief Description of the Drawings

Exemplary embodiments disclosed herein may be more completely understood in consideration of the following detailed description in connection with the following figures. The figures are not necessarily drawn to scale. Like numbers used in the figures refer to like components. However, it will be understood that the use of a number to refer to a component in a given figure is not intended to limit the component in another figure labeled with the same number.
FIG. 1 is a schematic block diagram of a system for determining structured data, according to an embodiment of the present disclosure.
FIG. 2 is a schematic block flow diagram of the system illustrating an example of a transcript of a conversation between a physician and a patient, according to an embodiment of the present disclosure.
FIG. 3 is a schematic block diagram of the system for training a machine learning model, according to an embodiment of the present disclosure; and
FIG. 4 is a flowchart illustrating a method for determining structured data, according to an embodiment of the present disclosure.

### Detailed Description

In the following description, reference is made to the accompanying figures that form a part thereof and in which various embodiments are shown by way of illustration. It is to be understood that other embodiments are contemplated and may be made without departing from the scope or spirit of the present disclosure. The following detailed description, therefore, is not to be taken in a limiting sense.

In the following disclosure, the following definitions are adopted.

As used herein, the term "patient", and its equivalents, may refer to an individual being monitored and/or cared for within a clinical environment or who has been previously monitored and/or cared for within the clinical environment. In various examples, a patient is a human, but implementations of this disclosure are not so limited. Clinical environment may include, but is not limited to, a doctor's office, a medical facility, a medical practice, a medical lab, an urgent care facility, a medical clinic, an emergency room, an operating room, a hospital, a long term care facility, a rehabilitation facility, a nursing home, and a hospice facility.

As used herein, the term "medical record" may refer to medical data generated by a provider for an individual person. The medical record may be in the form of a text document. The medical record may also be referred to as an electronic medical record (EMR).

As used herein, the term "provider" may refer to, for example, a physician (including, but not limited to, a radiologist, a surgeon, a primary care physician, and a medical specialist), a physician assistant, a nursing professional, a medical laboratory technician, medical clinics, hospitals, health insurance providers, diagnostic sites, imaging sites, pharmacies, and the like. The term "provider" may also refer to an academic institution, a government research laboratory, a non-profit entity, or a for-profit entity, such as a pharmaceutical, health insurance, biotechnology, wearable device, physiological monitoring, or medical device company.

As used herein, the term "physician" may refer to a health care provider or a medical professional, such as a doctor, a nurse, or other appropriate clinician.

As used herein, the term "structured data" generally refers to data that is organized into specific fields as part of a schema, with each field having a defined purpose. Non-limiting examples of such fields may include medications, exam results, vital signs, etc. For example, structured data may include formatted annotations conforming to a particular standard, e.g., a SOAP (Subjective, Objective, Assessment, and Plan) record or note.

As used herein, the term "machine-learning model" may refer to a computer model or a computer representation that may be tuned (e.g., trained) based on inputs to approximate unknown functions. For example, the machine-learning model may include one or more of vectorization machine-learning models, sequence-to-sequence models, transformer models, a decision tree (e.g., a gradient boosted decision tree), a linear regression model, a logistic regression model, association rule learning, inductive logic programming, support vector learning, a Bayesian network, a regression-based model, a neural network, or a combination thereof. The process of building or optimizing a machine learning model is referred to herein as "training".

As used herein, the term "neural network" may refer to one example of a machine learning model that can be tuned (e.g., trained) based on inputs to approximate unknown functions. In particular, the neural network may include a model of interconnected neurons (arranged in layers) that communicate and learn to approximate complex functions and generate outputs based on a plurality of inputs provided to the model. For example, the neural network may include deep convolutional neural networks (CNN), Region-CNN (R-CNN), Faster R-CNN, Mask R-CNN, fully convolutional neural networks, recurrent neural networks ("RNNs"), such as long short-term memory neural networks ("LSTMs"), graph neural networks, generative adversarial neural networks (GAN), and single-shot detect (SSD) networks. In other words, a neural network is an algorithm that implements deep learning techniques, which utilize a set of learned parameters arranged in layers according to a particular architecture to attempt to model high-level abstractions in data using supervisory data to tune parameters of the neural network.

As used herein, the term "coupled" generally means either a direct connection between two or more elements that are connected or an indirect connection through one or more passive or active intermediary devices.

As used herein, the term "communicably coupled" generally refers to any type of connection or coupling that allows for exchange or sharing of information. The term communicably coupled may include, but is not limited to, electrically coupled (e.g., through a wire), optically coupled (e.g., through an optical cable), wirelessly coupled (e.g., through a radio frequency or other similar technologies), and/or the like. The technology by which the information is transmitted is not material to the meaning of communicably coupled.

As used herein, all numbers should be considered modified by the term "about". As used herein, "a,'' "an," "the," "at least one," and "one or more" are used interchangeably.

The term "about", unless otherwise specifically defined, means to a high degree of approximation (e.g., within +/- 5% for quantifiable properties) but again without requiring absolute precision or a perfect match.

As used herein as a modifier to a property or attribute, the term "generally", unless otherwise specifically defined, means that the property or attribute would be readily recognizable by a person of ordinary skill but without requiring absolute precision or a perfect match (e.g., within +/- 20 % for quantifiable properties).

As used herein, the term "configured to" and like is at least as restrictive as the term "adapted to" and requires actual design intention to perform the specified function rather than mere physical capability of performing such a function.

Conversations between patients and medical practitioners, such as doctors and nurses, are often recorded. The record of the conversation (e.g., a clinical note), and a transcript, are a part of the patient's medical record data. The transcript may be generated by automatic speech recognition. For automating a process of generating the clinical note based on doctor-patient conversation transcript, pieces of information (e.g., words, phrases, etc.) may need to be extracted from the transcript. Such pieces of information may be combined with other pieces to form a target structed data. Structured data is data organized into specific fields as part of a schema, with each field having a defined purpose. Examples of such fields may include numbers, anatomical structures, laterality, etc. Structured data categories may include patient history, family history, past surgeries, medications, exam results, vital signs, and more. Common challenges in automated extraction of structured data from conversational text may include variety in vocabularies and sentence structures in the text, presence of medically irrelevant information, noise such as fillers, garbles, restating, repeating, rephrasing, etc., implicitly or contextually assumed information, unreliable sentence delimitation by the upstream automatic speech recognition, and pieces of information relevant to a single structured data instance but mentioned apart from each other.

The present disclosure provides a system for determining structured data. The system includes at least one non-transitory computer-readable storage medium having instructions stored thereon. The system further includes at least one computer processor coupled to the at least one non-transitory computer-readable storage medium and configured to execute the instructions to receive a plurality of medical concepts and a corresponding plurality of labels. Each medical concept from the plurality of medical concepts is associated with a corresponding label from the plurality of labels. The computer processor is further configured to receive a plurality of target attributes associated with a plurality of fields of one or more structured data. Each field from the plurality of fields is associated with one or more target attributes from the plurality of target attributes. The computer processor is further configured to receive a transcript of a conversation between a physician and a patient. The computer processor is further configured to determine a plurality of transcript concepts in the transcript based on the plurality of medical concepts. Each transcript concept from the plurality of transcript concepts is a corresponding medical concept from the plurality of medical concepts. The computer processor is further configured to assign each transcript concept with the label associated with the corresponding medical concept. The computer processor is further configured to determine a plurality of concept combinations by combining the plurality of transcript concepts. Each concept combination from the plurality of concept combinations is a combination of two or more transcript concepts from the plurality of transcript concepts, such that the labels of the two or more transcript concepts associate with one or more target attributes from the plurality of target attributes of a same field from the plurality of fields. The computer processor is further configured to determine, via a machine learning model, a combination label for each concept combination. The combination label is a valid label or an invalid label. The computer processor is further configured to generate an output structured data based on the concept combinations having the valid label.

The system of the present disclosure utilizes identification of the plurality of transcript concepts in the transcript of the conversation between the physician and the patient followed by determination of the plurality of concept combinations and the associated combination labels for extraction of the output structured data. Filtering of a text of the transcript with the plurality of transcript concepts may reduce computational resources required as compared to using the entire text for further processing. Combining the plurality of transcript concepts (to determine the plurality of concept combinations) regardless of their locations in the conversation overcomes the current challenge of linking distant pieces of information together. The machine learning model may allow classification of each concept combination to determine the associated combination label, thereby predicting if the concept combination forms a valid instance of the output structured data. Subsequently, the system may utilize the plurality of concept combinations having the combination label as the valid label for generating the output structured data.

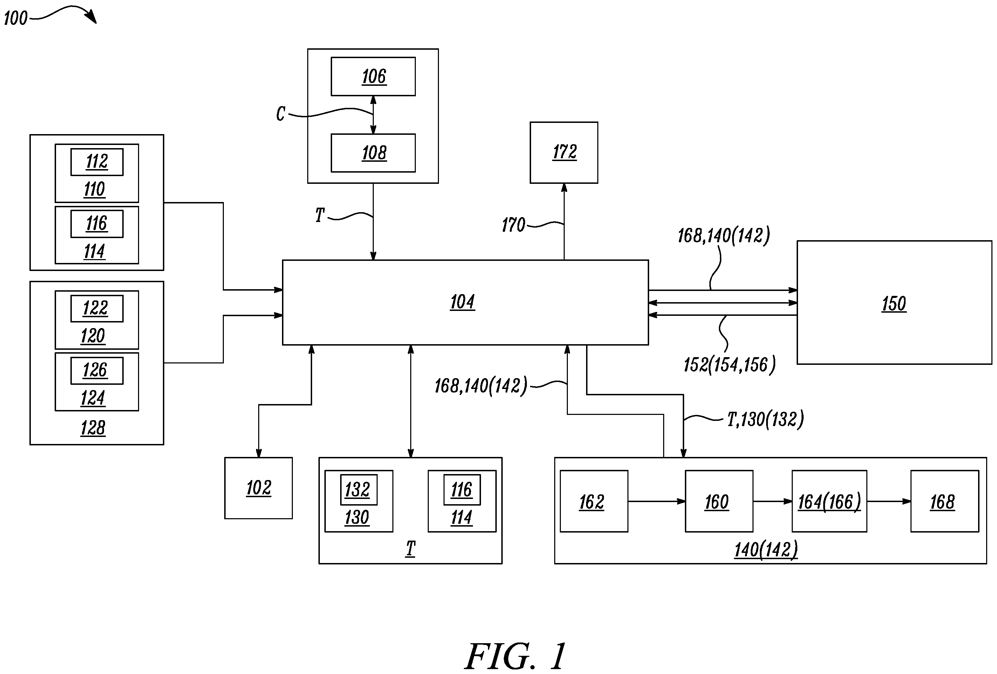

FIG. 1 is a schematic block diagram of a system 100 for determining structured data. In some embodiments, the structured data is data organized into specific fields as part of a schema, with each field having a defined purpose. In some embodiments, the structured data may be associated with a section of a medical record, e.g., medications, exam results, vital signs, patient history, family history, past surgeries, etc.

In some embodiments, the system 100 includes at least one non-transitory computer-readable storage medium 102 having instructions stored thereon. The system 100 further includes at least one computer processor 104 coupled to the at least one non-transitory computer-readable storage medium 102. The term "at least one non-transitory computer-readable storage medium 102" is interchangeably referred to herein as "the storage medium 102". The term "at least one computer processor 104" is interchangeably referred to herein as "the computer processor 104".

In some embodiments, the storage medium 102 may include any type of computer readable storage media, including, but not be limited to, various types of volatile and non-volatile storage media, including, but not limited to, random access memory, read-only memory, programmable read-only memory, electrically programmable read-only memory, electrically erasable read-only memory, flash memory, magnetic tape or disk, optical media, and the like. In some cases, the storage medium 102 may include a cache or random-access memory for the computer processor 104. Alternatively, or in addition, the storage medium 102 may be separate from the computer processor 104, such as a cache memory, a system memory, or other memory. In some embodiments, the storage medium 102 may be an external storage device or a database for storing data. Examples may include a hard drive, compact disc ("CD"), digital video disc ("DVD"), memory card, memory stick, floppy disc, universal serial bus ("USB") memory device, or any other device operative to store data.

In some embodiments, the computer processor 104 may be embodied in a number of different ways. For example, the computer processor 104 may be embodied as various processing means, such as one or more of a microprocessor or other processing elements, a coprocessor, or various other computing or processing devices, including integrated circuits, such as, e.g., an ASIC (application specific integrated circuit), an FPGA (field programmable gate array), or the like. As such, whether configured by hardware or by a combination of hardware and software, the computer processor 104 may represent an entity (e.g., physically embodied in circuitry - in the form of processing circuitry) capable of performing operations according to some embodiments while configured accordingly. Thus, for example, when the computer processor 104 is embodied as an executor of software instructions, the instructions may specifically configure the computer processor 104 to perform the operations described herein. Alternatively, as another example, when the computer processor 104 is embodied as an ASIC, FPGA, or the like, the computer processor 104 may have specifically configured hardware for conducting the operations described herein.

In some embodiments, the storage medium 102 may be communicatively coupled to the computer processor 104 by way of one or more wired and/or wireless communication interfaces. In some examples, the wireless communication interface may communicate data via one or more wireless communication protocols, such as BLUETOOTH, infrared, Wi-Fi, Zigbee, wireless universal serial bus (USB), radio frequency, near-field communication (NFC), RFID protocols, IEEE 802.11a, 802.11b, 802.11g, 802.11n, or generally any wireless communication protocol.

In some embodiments, the system 100 may be implemented as a server-side application, a client-side application, or a hybrid server-side/client-side application, and may be connected to a network (e.g., the Internet or a local area network). In some embodiments, the system 100 may include various components, examples of which may include, but are not limited to, a personal computer, a server computer, a series of server computers, a mini computer, a mainframe computer, one or more Network Attached Storage (NAS) systems, one or more Storage Area Network (SAN) systems, one or more Platform as a Service (PaaS) systems, one or more Infrastructure as a Service (IaaS) systems, one or more Software as a Service (SaaS) systems, a cloud-based computational system, and a cloud-based storage platform. In some embodiments, the system 100 may include a network module (not shown) that permits communication with other systems or computing devices, e.g., over a local area network or over the Internet.

For example, the system 100 may include a server or access to cloud infrastructure via the network to provide clinical documentation tools and/or access to electronic medical record (EMR) within a number of hospitals, hospital networks, other care facilities, or any other type of medical information system. The system 100 may have access to identification of patients being examined as well as access to their EMR. The system 100 may also have access to, e.g., notes from any medical staff that may have been entered in real time or that may have been previously entered.

In some embodiments, the network may include, but is not limited to, wired networks, wireless networks, and combined wired and wireless networks. For example, the network may include any type of network (including infrastructure) that provides communications, exchanges information, and/or facilitates exchange of information, such as the Internet, a private data network, a virtual private network using a public network, a local area network (LAN) or a wide area network (WAN), a Wi-Fi network, and/or other suitable connections that may enable information exchange among various components of the system 100. The network may also include a public switched telephone network (PSTN) and/or a wireless cellular network. The network may be a secured network or an unsecured network.

The at least one computer processor 104 is configured to execute the instructions to receive a plurality of medical concepts 110 and a corresponding plurality of labels 114. In some embodiments, the plurality of medical concepts 110 include multiple medical concepts 112. In some embodiments, the plurality of labels 114 include multiple labels 116. Each medical concept 112 from the plurality of medical concepts 110 is associated with a corresponding label 116 from the plurality of labels 114. The medical concepts 112 may include, e.g., anatomical locations, signs and symptoms, diagnoses, medications, referrals, investigations and therapies, reasons for visit, severity, location, frequency, time of onset of a symptom entity, etc. Such a vocabulary may be stored in the storage medium 102.

In some embodiments, the plurality of labels 114 may also be stored in the storage medium 102. In some embodiments, each label 116 may define a type of the corresponding medical concept 112. For example, "neck" or "knee" would be labeled as a "body structure", "five" would be labelled as a "number", etc. Specifically, each label 116 may encode the corresponding medical concept 112.

The at least one computer processor 104 is further configured to receive a plurality of target attributes 124 associated with a plurality of fields 120 of one or more structured data 128. In some embodiments, the plurality of target attributes 124 include multiple target attributes 126 and the plurality of fields 120 include multiple fields 122. Each field 122 from the plurality of fields 120 is associated with one or more target attributes 126 from the plurality of target attributes 124. In some embodiments, the plurality of target attributes 124 and the plurality of fields 120 of the one or more structured data 128 may be stored in the storage medium 102. In some embodiments, the one or more structured data 128 may include multiple kinds of medical records. In some embodiments, the one or more structured data 128 may include the EMR.

In some embodiments, the plurality of fields 120 may be associated with the EMR. In some embodiments, each field 122 may be defined by the one or more target attributes 126 associated with it. Specifically, the one or more target attributes 126 may determine data that may be populated in the corresponding field 122. For example, the EMR may include a SOAP note (Subjective, Objective, Assessment, and Plan note). The SOAP note is a widely used format for taking medical notes or summaries. The summaries collected in the SOAP note are digitized and stored in the EMR. The fields 122 associated with the SOAP note may include subjective information (S), objective observations (O), assessments data (A), and plan data (P). Subjective information (S) reported by a patient may include one or more of patient behavior, patient complaint, symptoms, progress from last encounter, problem, medical issues impacting or influencing patient's day-to-day routine, family history, medical history, social history, and so forth.

Objective observations (O) may include quantifiable and measurable data including lab results, x-rays, ultrasounds, other relevant diagnostic information, including electrocardiograms, physician's observations from physical examinations, and so forth. Assessments data (A) may include physician diagnoses made by interpreting the information given by the patient during the visit, observation of previous and new symptoms, clinical stability, and any synthesis of the subjective (S) and objective (O) sections made by the physician. Plan data (P) may include data indicative of plans for future care, e.g., indication of further investigation of the problem, diagnostic tests, investigated medications, treatments, follow-up protocol, and so forth. In some embodiments, other examples of the fields 122 may include a chief complaint section (e.g., a primary reason for a patient's visit), an allergies section, a past medical history section, and so forth.

The at least one computer processor 104 is further configured to receive a transcript T of a conversation C between a physician 106 and a patient 108. **In** some embodiment, the conversation C between the physician 106 and the patient 108 may be captured in the form of audio recording from their session, whether the session is held in-person or via tele-conferencing or video conferencing. **In** some embodiments, the transcript T of the conversation C may be obtained either by a trained transcriptionist or by use of a speech-to-text converter. **In** the latter case, the captured conversation may be transcribed using automatic speech recognition (ASR). **In** some embodiments, the transcript T may be stored in the storage medium 102.

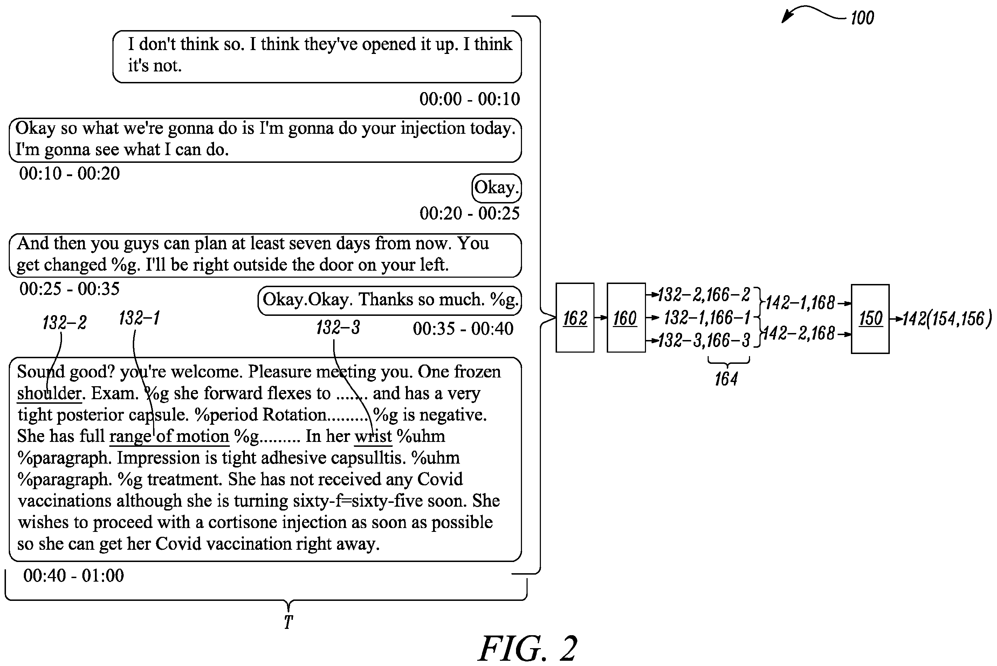

FIG. 2 is a schematic block flow diagram of the system 100 illustrating an example of the transcript T. In some embodiments, the transcript T may be preferably accompanied by a time indexing, in which words spoken in the transcript T or lines of text are associated with elapsed time of the conversation C.

Referring to FIGS. 1 and 2, the at least one computer processor 104 is further configured to determine a plurality of transcript concepts 130 in the transcript T based on the plurality of medical concepts 110. The plurality of transcript concepts 130 may include multiple transcript concepts 132-1, 132-2, ..., 132-N (collectively, transcript concepts 132), where N is a positive integer corresponding to a total number of the transcript concepts 132 in the plurality of transcript concepts 130.

Each transcript concept 132 from the plurality of transcript concepts 130 is a corresponding medical concept 112 from the plurality of medical concepts 110. In some embodiments, the computer processor 104 may identify the plurality of transcript concepts 130 (or the plurality of medical concepts 110) in spans of text of the transcript T. For example, the computer processor 104 may identify noteworthy utterances that are relevant for specific fields 122 of the one or more structured data 128, e.g., medical history, surgical history, allergies, etc.

As shown in FIG. 2, the terms "range of motion", "shoulder", "wrist" may be identified as transcript concepts 132-1, 132-2, 132-3, respectively, in the transcript T. In some embodiments, the at least one computer processor 104 is further configured to annotate the transcript T with the plurality of transcript concepts 130. For example, the at least one computer processor 104 may highlight the spans of text in the transcript T corresponding to the transcript concepts 132.

The at least one computer processor 104 is further configured to assign each transcript concept 132 with the label 116 associated with the corresponding medical concept 112. In some embodiments, the labels 116 associated with the transcript concepts 132 may be utilized to form a group of the transcript concepts 132 that form an instance of a structured data for populating a corresponding field 122. For example, the plurality of labels 114 may be associated with the one or more target attributes 126 of the corresponding field 122. In an example, the field 122 associated with "exam results" may require inputs such as a body structure, a type of exam, and a number or a range. The body structure, the type of exam, and the number may be labels 116 associated with the transcript concepts 132 that need to be identified in the transcript T.

The at least one computer processor 104 is further configured to determine a plurality of concept combinations 140 by combining the plurality of transcript concepts 130. In some embodiments, the plurality of concept combinations 140 include multiple concept combinations 142-1, 142-2, ..., 142-M (collectively, concept combinations 142), where M is a positive integer corresponding to a total number of the concept combinations 142 in the plurality of concept combinations 140. Each concept combination 142 from the plurality of concept combinations 140 is a combination of two or more transcript concepts 132 from the plurality of transcript concepts 130, such that the labels 116 of the two or more transcript concepts 132 associate with one or more target attributes 126 from the plurality of target attributes 124 of a same field 122 from the plurality of fields 120. In other words, each concept combination 142 may include the two or more transcript concepts 132 with corresponding labels 116 that align with the one or more target attributes 126 of the same field 122, such that the two or more transcript concepts 132 may form an instance of a structured data that may populate the corresponding field 122. In some embodiments, each concept combination 142 may represent relatedness of the corresponding two or more transcript concepts 132 as they are related to a same medical condition of the patient 108.

In some embodiments, for each concept combination 142, the at least one computer processor 104 is further configured to generate one or more text encodings 160. In some embodiments, the one or more text encodings 160 may include multiple text encodings 160. Each of the one or more text encodings 160 is generated by encoding at least a portion of the transcript T including one or more transcript concepts 132 from the plurality of transcript concepts 130. In some embodiments, the one or more transcript concepts 132 include multiple transcript concepts 132. Each text encoding 160 containing the portion of the transcript T may also contain contextual information associated with the one or more transcript concepts 132 that are included in the portion of the transcript T. Thus, in some embodiments, portions of the transcript T may also be used without any overlap to determine each concept combination 142 based on the text encodings 160.

In some embodiments, the one or more text encodings 160 may be generated by a text encoder 162 using a pretrained language model, e.g., Word2vec, Long Short-Term Memory (LSTM), Transformer, etc. For example, the text encoder 162 may encode the portion of the transcript T including the one or more transcript concepts 132 in the form of the one or more text encodings 160. Specifically, the text encoder 162 may transform the portion of the transcript T (text) into a numerical representation. As shown in FIG. 2, the concept combination 142-1 incudes the transcript concepts 132-1, 132-2, i.e., "range or motion" and "shoulder". Further, the concept combination 142-2 includes the transcript concepts 132-1, 132-3, i.e., "range or motion" and "wrist".

In some embodiments, for each concept combination 142, the at least one computer processor 104 is further configured to determine a plurality of slices 164 of a tensor representation of the one or more text encodings 160 corresponding to the one or more transcript concepts 132. In some embodiments, the plurality of slices 164 include multiple slices 166-1, 166-2, ..., 166-P (collectively, slices 166), where P is a positive integer corresponding to a total number of the slices 166 in the plurality of slices 164.

As used herein, the term "tensor" generally refers to a linear geometrical quantity. In the present description, the tensor is typically in the dimension of "[token, dims]", where "dims" is a length of word vectors that the text encoder (e.g., the text encoder 162) in use employs. Creating a slice means taking a portion of the tensor at a token ID corresponding to the transcript concept and obtaining the [dims] vector. This contains semantic and contextual information about a specific mention of the transcript concept.

Utilizing encodings that contain contextual information is particularly useful in medical conversational settings. Such advantage can be seen in a case where relevant information is spread across the breadth of the conversation. For instance, in a visit where the patient describes the pain in their shoulder at the beginning of the conversation, the doctor may not explicitly mention the anatomical structure evaluated later in the conversation, because it is naturally implied to be "shoulder." Contextual information contained in encodings provides the opportunity to link the mention of "shoulder" occurred at the beginning of the conversation to the evaluation occurred later in the conversation. The advantage can also be seen in another case where the particular use or use case can lead to a different interpretation of the surrounding language. For instance, the word "head" in the patient's utterance "my head has been hurting" may imply a head injury, whereas the same word "head" in the doctor's utterance "turn your head" may imply an instruction during an investigation, or a mention of "the head of the femur" may relate to an entirely different anatomical region. Contextual information contained in encodings provides the opportunity to distinguish between mentions of "head" and identify the attributes of interest that can be reasonably assigned. In other words, relying on contextual information can result in improved accuracy because the systems and techniques described herein that utilize contextual information as described can identify more relevant attributes of interest more often than systems that are agnostic to this contextual information.

Each slice 166 from the plurality of slices 164 contains a corresponding transcript concept 132 from the one or more transcript concepts 132. As shown in FIG. 2, the slice 166-1 contains the transcript concept 132-1, the slice 166-2 contains the transcript concept 132-2, and the slice 166-3 contains the transcript concept 132-3. Each slice 166 from the plurality of slices 164 represents a portion of the tensor representation of the one or more text encodings 160 at the corresponding transcript concept 132 from the one or more transcript concepts 132. In other words, each slice 166 may represent a portion of the numerical representation of the one or more text encodings 160 that includes the corresponding transcript concept 132 from the one or more transcript concepts 132.

In some embodiments, for each concept combination 142, the at least one computer processor 104 is further configured to determine a tensor 168 by concatenating the plurality of slices 164 corresponding to the one or more transcript concepts 132. In other words, the computer processor 104 may concatenate the numerical representations corresponding to the one or more transcript concepts 132 to determine the tensor 168. In some embodiments, the tensor 168 may be a flat tensor with a single dimension.

In some embodiments, the at least one computer processor 104 is further configured to feed the tensors 168 corresponding to the plurality of concept combinations 140 to the machine learning model 150. In some embodiments, the machine learning model 150 may include a neural network, however, other machine learning frameworks may also be utilized. The at least one computer processor 104 is further configured to determine, via the machine learning model 150, a combination label 152 for each concept combination 142. The combination label 152 is a valid label 154 or an invalid label 156.

In some embodiments, the machine learning model 150 is a binary classifier model. The machine learning model 150 may predict the combination label 152 as the valid label 154 or the invalid label 156. In other words, the machine learning model 150 may predict whether the concept combination 142 including the two or more transcript concepts 132 is a valid combination or not. In some embodiments, inputs to the machine learning model 150 may also include contextual information from the transcript T for accurate prediction of the combination label 152.

The at least one computer processor 104 is further configured to generate an output structured data 170 based on the concept combinations 142 having the valid label 154. In other words, the output structured data 170 may include the two or more transcript concepts 132 associated with corresponding concept combination 142 having the valid label 154. Thus, the system 100 may extract clinically relevant information from the transcript T and assist in organizing medical information and generating medical records.

In some embodiments, the at least one computer processor 104 is further configured to output the output structured data 170 to at least one of a user interface 172 and the at least one non-transitory computer-readable storage medium 102. In some embodiments, the user interface 172 may be accessible to the physician 106 for providing confirmation and feedback. The storage medium 102 may store the output structured data 170 for subsequent generation of the medical record.

In some embodiments, the at least one computer processor 104 is further configured to link the output structured data 170 with the plurality of transcript concepts 130 in the transcript T. For example, the plurality of transcript concepts 130 may be linked to the output structured data 170 for evidence linking. This may allow the plurality of transcript concepts 130 that are associated with the output structured data 170 to be reviewed and verified.

In some embodiments, the at least one computer processor 104 is further configured to visually highlight the plurality of transcript concepts 130 in the transcript T linked to the output structured data 170. In one example, when a user of the system 100 hovers a pointer or a cursor over the output structured data 170 in the user interface 172, the corresponding transcript concepts 132 may get highlighted in the transcript T, e.g., via a pop up. In another example, the computer processor 104 may color code the plurality of transcript concepts 130 in the transcript T in a same color as that of the output structured data 170.

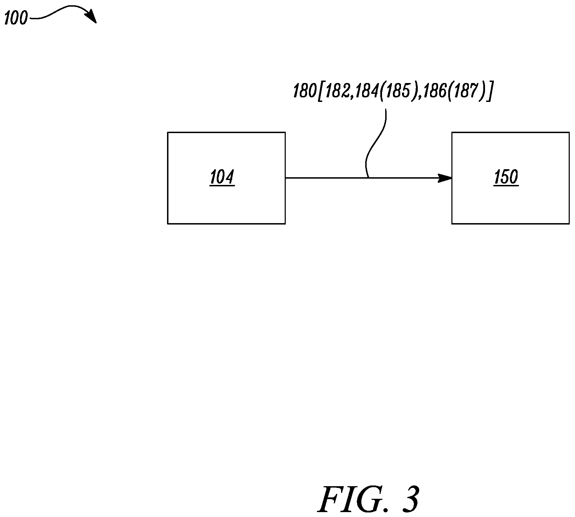

FIG. 3 is a schematic block diagram of the system 100 for training the machine learning model 150, according to an embodiment of the present disclosure. In some embodiments, the at least one computer processor 104 is further configured to train the machine learning model 150 using a training data 180 (or ground truth dataset) including a training transcript 182, a plurality of training concept combinations 184, and a plurality of training combination labels 186 corresponding to the plurality of training concept combinations 184. In some embodiments, the training transcript 182 of the training data 180 may be annotated with predefined concepts, along with labels representing sets of concepts that form an instance of a target structured data.

In some embodiments, the plurality of training concept combinations 184 include multiple training concept combinations 185. Further, the plurality of training combination labels 186 include multiple training combination labels 187. Each training concept combination 185 may include two or more training concepts. Further, each training combination label 187 may indicate if the corresponding training concept combination 185 is valid or invalid.

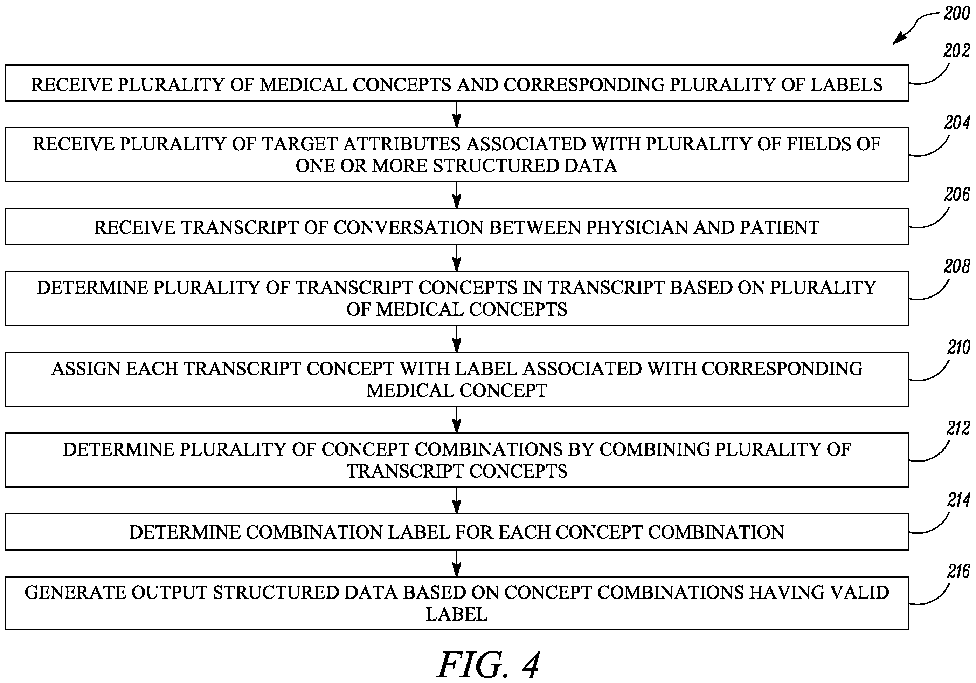

FIG. 4 is a flowchart illustrating a method 200 for determining structured data. The method 200 may be implemented using the system 100 of FIGS. 1-3. Referring now to FIGS. 1-4, at step 202, the method 200 includes receiving, via the at least one computer processor 104, the plurality of medical concepts 110 and the corresponding plurality of labels 114. Each medical concept 112 from the plurality of medical concepts 110 is associated with the corresponding label 116 from the plurality of labels 114.

At step 204, the method 200 further includes receiving, via the at least one computer processor104, the plurality of target attributes 124 associated with the plurality of fields 120 of the one or more structured data 128. Each field 122 from the plurality of fields 120 is associated with the one or more target attributes 126 from the plurality of target attributes 124.

At step 206, the method 200 further includes receiving, via the at least one computer processor 104, the transcript T of the conversation C between the physician 106 and the patient 108.

At step 208, the method 200 further includes determining, via the at least one computer processor 104, the plurality of transcript concepts 130 in the transcript T based on the plurality of medical concepts 110. Each transcript concept 132 from the plurality of transcript concepts 130 is the corresponding medical concept 112 from the plurality of medical concepts 110. In some embodiments, the method 200 further includes annotating the transcript T with the plurality of transcript concepts 130.

At step 210, the method 200 further includes assigning, via the at least one computer processor 104, each transcript concept 132 with the label 116 associated with the corresponding medical concept 112.

At step 212, the method 200 further includes determining, via the at least one computer processor 104, the plurality of concept combinations 140 by combining the plurality of transcript concepts 130. Each concept combination 142 from the plurality of concept combinations 140 is a combination of the two or more transcript concepts 132 from the plurality of transcript concepts 130, such that the labels 116 of the two or more transcript concepts 132 associate with the one or more target attributes 126 from the plurality of target attributes 124 of the same field 122 from the plurality of fields 120.

In some embodiments, the method 200 further includes generating the one or more text encodings 160. Each of the one or more text encodings 160 is generated by encoding at least a portion of the transcript T including the one or more transcript concepts 132 from the plurality of transcript concepts 130. In some embodiments, the method 200 further includes determining the plurality of slices 164 of a tensor representation of the one or more text encodings 160 corresponding to the one or more transcript concepts 132. Each slice 166 from the plurality of slices 166 contains the corresponding transcript concept 132 from the one or more transcript concepts 132. In some embodiments, the method 200 further includes determining the tensor 168 by concatenating the plurality of slices 164 corresponding to the one or more transcript concepts 132. In some embodiments, the method 200 further includes feeding the tensors 168 corresponding to the plurality of concept combinations 140 to the machine learning model 150.

At step 214, the method 200 further includes determining, via the machine learning model 150, the combination label 152 for each concept combination 142. The combination label 152 is the valid label 154 or the invalid label 156. In some embodiments, the machine learning model 150 is a binary classifier model.

At step 216, the method 200 further includes generating, via the at least one computer processor 104, the output structured data 170 based on the concept combinations 142 having the valid label 154. In some embodiments, the method 200 further includes outputting the output structured data 170 to at least one of the user interface 172 and the at least one non-transitory computer-readable storage medium 102. In some embodiments, the method 200 further includes linking the output structured data 170 with the plurality of transcript concepts 130 in the transcript T. In some embodiments, the method 200 further includes visually highlighting the plurality of transcript concepts 130 in the transcript T linked to the output structured data 170.

In some embodiments, the method 200 further includes training the machine learning model 150 using the training data 180 including the training transcript 182, the plurality of training concept combinations 184, and the plurality of training combination labels 186 corresponding to the plurality of training concept combinations 184.

The system 100 and the method 200 of the present disclosure utilizes identification of the plurality of transcript concepts 130 in the transcript T of the conversation C between the physician 106 and the patient 108 followed by determination of the plurality of concept combinations 140 and the associated combination labels 152 for extraction of the output structured data 170. Filtering of a text of the transcript T with the plurality of transcript concepts 130 may reduce computational resources required as compared to using the entire text of the transcript T for further processing.

Combining the plurality of transcript concepts 130 (to determine the plurality of concept combinations 140) regardless of their locations in the conversation C overcomes the current challenge of linking distant pieces of information together. The machine learning model 150 may allow classification of each concept combination 142 to determine the associated combination label 152, thereby predicting if the concept combination 142 forms a valid instance of the output structured data 170. Use of the one or more text encodings 160 may provide the capability to capture contextual information. Subsequently, the system 100 and the method 200 may utilize the plurality of concept combinations 140 having the combination label 152 as the valid label 154 for generating the output structured data 170.

Unless otherwise indicated, all numbers expressing feature sizes, amounts, and physical properties used in the specification and claims are to be understood as being modified by the term "about". Accordingly, unless indicated to the contrary, the numerical parameters set forth in the foregoing specification and attached claims are approximations that can vary depending upon the desired properties sought to be obtained by those skilled in the art utilizing the teachings disclosed herein.

As used in this specification and the appended claims, the singular forms "a," "an," and "the" encompass embodiments having plural referents, unless the content clearly dictates otherwise. As used in this specification and the appended claims, the term "or" is generally employed in its sense including "and/or" unless the content clearly dictates otherwise.

Spatially related terms, including but not limited to, "proximate," "distal," "lower," "upper," "beneath," "below," "above," and "on top," if used herein, are utilized for ease of description to describe spatial relationships of an element(s) to another. Such spatially related terms encompass different orientations of the device in use or operation in addition to the particular orientations depicted in the figures and described herein. For example, if an object depicted in the figures is turned over or flipped over, portions previously described as below, or beneath other elements would then be above or on top of those other elements.

As used herein, when an element, component, or layer for example is described as forming a "coincident interface" with, or being "on," "connected to," "coupled with," "stacked on" or "in contact with" another element, component, or layer, it can be directly on, directly connected to, directly coupled with, directly stacked on, in direct contact with, or intervening elements, components or layers may be on, connected, coupled or in contact with the particular element, component, or layer, for example. When an element, component, or layer for example is referred to as being "directly on," "directly connected to," "directly coupled with," or "directly in contact with" another element, there are no intervening elements, components or layers for example.

It should be appreciated that the innovations disclosed herein are generally adapted to configure computer-based systems to address technical problems related to determining structured data, and in particular, the technical problem of determining structured data from conversational speech. That is, the innovations described herein are inextricably linked to computing systems that generate structured data from conversations (e.g., audio or transcription-based conversations). For instance, according to particular implementations, receiving a plurality of medical concepts and a corresponding plurality of labels may involve billions of separate calculations. **In** particular, when utilizing a machine learning model as contemplated by the instant disclosure to generate the plurality of medical concepts and the corresponding plurality of labels that are received by systems of the instant disclosure, each concept must be processed based on the number of parameters (or weights) of the model. For the model to be sufficiently accurate, the number of parameters of the model must be large. For instance, the GPT-3 model utilizes a model with more than 100 billion parameters. In other words, using a GPT-3 model (or something similarly sized), each concept must be processed using more than 100 billion discrete calculations. Similarly, when training a machine learning model, the model must be trained in a forward pass of computations, and a backward pass of computations (known as backpropagation), until the model converges after a certain (but generally unknown) number of epochs (or iterations). As a result, even when the machine learning model to be trained according to this disclosure is fractionally the size of a large model (such as the GPT-3 model), training the model for its intended purpose as disclosed herein will nevertheless involve millions, or even billions, of calculations. In short, the sheer magnitude of the parameters and calculations involved for each concept takes the disclosed techniques outside the realm of what is practicable for a human to perform (either in the mind of a human or with the aid of pen and paper) and inextricably links the technology to the realm of computers.

In addition, the described system generally must act in a time-constrained manner to achieve its desired results. For instance, in the context of a doctor-patient conversation, insights into those conversations are only functionally useful in the short window between when the conversation has happened and the time the conversation must be documented. For example, if a doctor has a 20-minute conversation with the patient, exam results and other clinical concepts need to be available essentially immediately to allow the captured information to be entered into the medical record or perform outcome planning. As a result, systems and techniques disclosed herein must be capable of operating in real-time or near real-time.

If, for example, this work was completed by hand and the aggregation of medical concepts was done via the same techniques disclosed herein, it could take potentially months to return the concepts to the physician, therefore rendering the concepts largely useless as they would no longer be actionable due to a change in the patient's status, medical condition, and the like. While concepts such as billing codes, can have more delay between the end of the encounter and when the computed result is returned, clinical concepts are largely viewed exclusively by the doctor, which can help achieve improved medical outcomes. This means that they need to be completed before the doctor completes charting on a given patient which often occurs during the course of the immediate or subsequent workday after the doctor patient/encounter. In contrast, given the large volume of data needed to utilize the techniques disclosed herein, it would take a human many weeks or months to generate the embedding for each identified concept, let alone statistically analyze each embedding pair making this approach without a machine learning model impracticable to perform by a human with or without the aid of pen and paper.

Various examples have been described. These and other examples are within the scope of the following claims.

## Claims

1. A system for determining structured data, the system comprising:
at least one non-transitory computer-readable storage medium having instructions stored thereon; and
at least one computer processor coupled to the at least one non-transitory computer-readable storage medium and configured to execute the instructions, in real-time or near real-time, to:
receive a plurality of medical concepts and a corresponding plurality of labels, wherein each medical concept from the plurality of medical concepts is associated with a corresponding label from the plurality of labels;
receive a plurality of target attributes associated with a plurality of fields of one or more structured data, wherein each field from the plurality of fields is associated with one or more target attributes from the plurality of target attributes;
receive a transcript of a conversation between a physician and a patient;
determine a plurality of transcript concepts in the transcript based on the plurality of medical concepts, wherein each transcript concept from the plurality of transcript concepts is a corresponding medical concept from the plurality of medical concepts;
assign each transcript concept with the label associated with the corresponding medical concept;
determine a plurality of concept combinations by combining the plurality of transcript concepts, wherein each concept combination from the plurality of concept combinations is a combination of two or more transcript concepts from the plurality of transcript concepts, such that the labels of the two or more transcript concepts associate with one or more target attributes from the plurality of target attributes of a same field from the plurality of fields;
determine, via a machine learning model, a combination label for each concept combination, the combination label being a valid label or an invalid label; and
generate an output structured data based on the concept combinations having the valid label.

2. The system of claim 1, wherein, for each concept combination, the at least one computer processor is further configured to:
generate one or more text encodings, wherein each of the one or more text encodings is generated by encoding at least a portion of the transcript comprising one or more transcript concepts from the plurality of transcript concepts;
determine a plurality of slices of a tensor representation of the one or more text encodings corresponding to the one or more transcript concepts, wherein each slice from the plurality of slices contains a corresponding transcript concept from the one or more transcript concepts; and
determine a tensor by concatenating the plurality of slices corresponding to the one or more transcript concepts.

3. The system of any of claim 1-2, wherein the at least one computer processor is further configured to feed the tensors corresponding to the plurality of concept combinations to the machine learning model.

4. The system of any of claims 1-3, wherein the machine learning model is a binary classifier model.

5. The system of any of claims 1-4, wherein the at least one computer processor is further configured to train the machine learning model using a training data comprising a training transcript, a plurality of training concept combinations, and a plurality of training combination labels corresponding to the plurality of training concept combinations.

6. The system of any of claims 1-5, wherein the at least one computer processor is further configured to annotate the transcript with the plurality of transcript concepts.

7. The system of any of claims 1-6, wherein the at least one computer processor is further configured to output the output structured data to at least one of a user interface and the at least one non-transitory computer-readable storage medium.

8. The system of any of claims 1-7, wherein the at least one computer processor is further configured to link the output structured data with the plurality of transcript concepts in the transcript.

9. The system of any of claims 1-8, wherein the at least one computer processor is further configured to visually highlight the plurality of transcript concepts in the transcript linked to the output structured data.

10. A method for determining structured data, the method comprising:
receiving, in real-time and via at least one computer processor, a plurality of medical concepts and a corresponding plurality of labels, wherein each medical concept from the plurality of medical concepts is associated with a corresponding label from the plurality of labels;
receiving, in real-time and via the at least one computer processor, a plurality of target attributes associated with a plurality of fields of one or more structured data, wherein each field from the plurality of fields is associated with one or more target attributes from the plurality of target attributes;
receiving, in real-time and via the at least one computer processor, a transcript of a conversation between a physician and a patient;
determining, in real-time and via the at least one computer processor, a plurality of transcript concepts in the transcript based on the plurality of medical concepts, wherein each transcript concept from the plurality of transcript concepts is a corresponding medical concept from the plurality of medical concepts;
assigning, in real-time and via the at least one computer processor, each transcript concept with the label associated with the corresponding medical concept;
determining, in real-time and via the at least one computer processor, a plurality of concept combinations by combining the plurality of transcript concepts, wherein each concept combination from the plurality of concept combinations is a combination of two or more transcript concepts from the plurality of transcript concepts, such that the labels of the two or more transcript concepts associate with one or more target attributes from the plurality of target attributes of a same field from the plurality of fields;
determining, in real-time and via a machine learning model, a combination label for each concept combination, the combination label being a valid label or an invalid label; and
generating, in real-time and via the at least one computer processor, an output structured data based on the concept combinations having the valid label.

11. The method of claim 10, wherein, for each concept combination, the method further comprises:
generating one or more text encodings, wherein each of the one or more text encodings is generated by encoding at least a portion of the transcript comprising one or more transcript concepts from the plurality of transcript concepts;
determining a plurality of slices of a tensor representation of the one or more text encodings corresponding to the one or more transcript concepts, wherein each slice from the plurality of slices contains a corresponding transcript concept from the one or more transcript concepts; and
determining a tensor by concatenating the plurality of slices corresponding to the one or more transcript concepts.

12. The method of any of claims 10-11, further comprising feeding the tensors corresponding to the plurality of concept combinations to the machine learning model.

13. The method of any of claims 10-12, wherein the machine learning model is a binary classifier model.

14. The method of any of claims 10-13, further comprising training the machine learning model using a training data comprising a training transcript, a plurality of training concept combinations, and a plurality of training combination labels corresponding to the plurality of training concept combinations.

15. The method of any of claims 10-14, further comprising annotating the transcript with the plurality of transcript concepts.
